# EUROPEAN PATENT APPLICATION

(11) **EP 2 218 419 A1**
(43) Date of publication of application: **18.08.2010**
(21) Application number: 08855961.2
(22) Date of filing: 02.12.2008
(51) Int. Cl.: A61B 18/20, A61B 5/151

(54) **LASER UNIT**

(30) Priority: 04.12.2007 JP 2007313273
(71) Applicant: Panasonic Corporation, Kadoma-shi Osaka 571-8501 (JP)
(72) Inventor: NISHIDA, Takeshi, 2-1-61, Shiromi Chuo-ku, Osaka 540-6207 (JP)
(74) Representative: Grünecker, Kinkeldey, Stockmair & Schwanhäusser Anwaltssozietät
(86) International application number: PCT/JP2008/003562
(87) International publication number: WO 2009/072270

(57) **Abstract**

It is possible to provide a laser unit which can sting a skin with a small-diameter hole causing a small pain. The laser unit (1) includes: a laser rod (3) which applies a laser beam; a lens (7) which collects a laser beam applied from an irradiation unit; and a laser transmission plate (9) having a mirror portion on the side to which the laser beam comes in and a rough portion having an average surface roughness Ra on the side from which the laser beam goes out. With this configuration, the laser beam which has passed through the laser transmission plate (9) is scattered by the rough portion and the intensity of the laser beam applied to the skin is not concentrated to the center. Moreover, the laser beam generates an impulse wave when passing through the rough portion. That is, the energy is discharged in advance.

## Description

### Technical Field

The present invention relates to a laser unit.

### Background Art

Lasers have spread rapidly and used widely in recent years in fields such as processing, medical care and measurement. As for kinds of lasers, there are solid lasers, gas lasers, fiber lasers, free electron lasers and so forth. In addition, laser oscillation modes include the continuous wave (CW) mode, the pulse mode and so forth, and their pulse widths and wavelengths can be arbitrarily set.

Among these lasers, a solid laser will be described. A solid laser is apparatus to capture laser light by illuminating a solid laser rod provided in a laser unit with excitation light. Laser excitation with ruby by Maiman is the beginning of a solid laser. In addition, the solid laser using YAG (acronym of yttrium, aluminum and garnet) as the rod base material is well known (see Patent Document 1**,** for example).

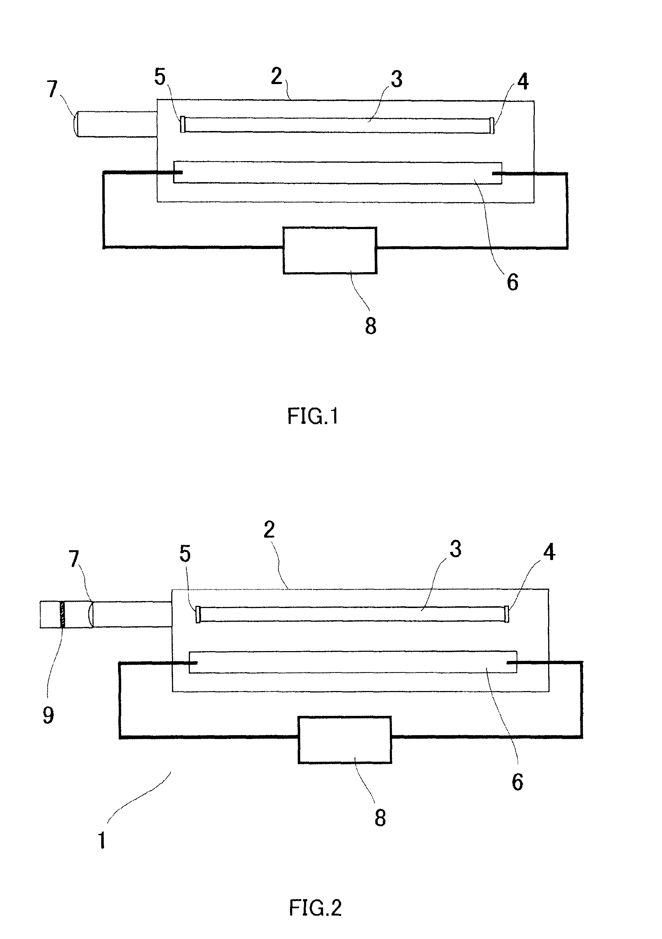

FIG.1 is a cross sectional view of a conventional solid laser unit.

As shown in FIG.1, the solid laser unit basically has: laser rod 3 that oscillates laser light; reflecting mirror 4 and reflecting mirror 5 for amplifying oscillated laser light; flash lamp 6 for illuminating laser rod 3 with light energy; power supply 8 for making flash lamp 6 emit light; and lens barrel 2 that covers flash lamp 6 entirely and reflects light to illuminate laser rod 3 with light.

Reflecting films may be formed on end surfaces of laser rod 3 instead of reflecting mirror 4 and reflecting mirror 5. The reflecting films are dielectric multilayer films and so forth, and are formed by a method such as deposition. The reflectivity of reflecting mirror 5 is set to 80 % to 95 %, and the reflectivity of reflecting mirror 4 is set equal to or more than 99.5 % (that is, nearly total reflection), so that it is possible to amplify laser light. Moreover, while laser light emitted from laser rod 3 is focused on one point with lens 7 placed on the line in the exit direction of laser rod 3 to increase the intensity, skin is placed in the vicinity of the focal point of the lens and punctured, so that blood spouts out.

When skin is punctured using this laser unit, laser light preferably has a wavelength readily absorbed by water, to prevent laser light from penetrating into skin, and the wavelength is preferably around 3 µm or 10µm.
Patent Document 1: Japanese Patent Application Laid-Open No.7-115237

### Disclosure of Invention

### Problems to be Solved by the Invention

When laser puncturing is performed, laser light illuminating skin evaporates moisture in a moment by oscillating the O-H group of moisture. At this time, skin cells is evaporated along with moisture and blood vessels are damaged at the same time, so that blood spouts out.

However, energy not used for evaporation penetrates into deep part of skin and stimulates the pain spot, which the patient feels as pain. As for the relationship between this pain and the incident angle of laser light on skin, experiments have shown that when the incident angle is larger, that is, when the focal distance is shorter, pain increases, and there is otherwise no pain. The energy required for evaporation is defined by laser output/ the area of the puncture diameter (referred to as "fluence"). To keep this fluence the same and make laser output lower is allowed by making the puncture diameter smaller.

Therefore, it is desirable to increase the focal distance of the lens to prevent pain and reduce the puncture diameter to save energy. However, there is a problem that if the focal distance is increased, due to the wave nature of laser light, it is not possible to make the laser spot diameter equal to or less than a certain size, and therefore, it is not possible to realize a laser unit to allow puncturing of a small laser spot diameter without pain.

It is therefore an object of the present invention to provide a laser unit allowing puncturing of a small puncture diameter with little pain.

### Means for Solving the Problem

The laser unit has a configuration including: an emitting section that emits laser light; a lens that focuses laser light emitted from the emitting section; and a laser transmitting plate placed on an optical path for emitting laser light, a surface receiving the laser light being a mirror surface, and a surface outputting the laser light having predetermined roughness.

### Advantageous Effects of Invention

According to the present invention, a laser unit being capable of puncturing of a small puncture diameter with little pain can be realized by placing a laser light transmitting plate in which the surface receiving laser light is a mirror surface and the surface outputting laser light has predetermined roughness in an optical path for emitting laser light.

That is, the peripheral part of laser light having passed through the laser light transmitting plate scatters, and therefore the intensity of laser light illuminating an object to illuminate is not concentrated at the center. Therefore, the scale of laser light is adjusted to a predetermined scale. Moreover, shock wave is generated when laser light passes through the part having predetermined roughness from the mirror side of the laser light transmitting plate and released as energy in the form of the shock wave in advance, so that, when the object to illuminate is skin (a finger, in particular) and so forth, a laser unit with little pain can be provided.

### Brief Description of Drawings

FIG.1 is a cross sectional view showing a conventional laser unit;
FIG.2 is a cross sectional view of a laser unit according to an embodiment of the present invention;
FIG.3 is a cross sectional view of a laser light transmitting plate of the laser unit according to the embodiment;
FIG.4A is a cross sectional view of the laser light transmitting plate of the laser unit according to the embodiment;
FIG.4B is a plan view of the laser light transmitting plate of the laser unit according to the embodiment;
FIG.5 is a cross sectional view of a flash lamp of the laser unit according to the embodiment;
FIG.6 is an explanatory drawing showing an operating state of the laser unit according to the embodiment;
FIG.7A is an explanatory drawing showing the performance of the laser unit according to the embodiment;
FIG.7B is an explanatory drawing showing the performance of the laser unit according to the embodiment;
FIG.8 is a drawing explaining a state of scattering of evaporated materials, which are generated by ablation when skin is illuminated with laser light from the laser unit according to the embodiment;
FIG.9 is a configuration diagram showing the laser unit according to the embodiment;
FIG.10 is a configuration diagram showing the laser unit according to the embodiment;
FIG.11 is a drawing a table showing a summary of an experimental apparatus applied to the laser unit according to the embodiment; and
FIG.12 is a drawing showing the degree of pain by puncturing with the laser unit produced according to the experimental apparatus of FIG.11.

### Best Mode for Carrying Out the Invention

Now, embodiments of the present invention will be described in detail with reference to the accompanying drawings.

First, the basic configuration of the present invention will be described.

The laser unit according to the present invention has a laser emitting section that emits laser light; a lens that focuses laser light emitted from the laser emitting section; and a laser transmitting plate in which the surface receiving laser light is a mirror surface and the surface outputting laser light has a predetermined roughness.

### [Laser emitting section]

Although a gas laser, a solid laser and a laser excited by a semiconductor laser are applicable as the laser emitting section as long as its laser wavelength can be readily absorbed by water, a case in which the solid laser is used will be described here.

The solid laser is composed of a laser rod doped with rare earth ions or transition metal ions such as erbium (Er), neodymium (Nd) or holmium (Ho), which are laser-active species; a flash lamp for emitting light to excite those laser-active species; and a lens barrel for efficiently focusing light from the flash lamp on the laser rod without leaking light outside. Exemplary solid lasers include a ruby laser, a glass laser, a YAG laser and so forth.

Assuming that the object to illuminate with laser light is human skin and is punctured, the laser rod is preferably a rod configured by a YAG laser doped with erbium (Er) at about 50 % A YAG laser may be configured by YAG single crystal or YAG ceramics. YAG ceramics are polycrystalline substance formed by coagulating YAG microcrystals.

The laser emitting section placed in the laser unit according to the present invention is required to have features for puncturing skin. That is, the diameter of laser emission before the laser light is incident on the lens is preferably about 0.5 mm to 3 mm, and the output of laser light is preferably about 30 mJ to 200 mJ.

A single-crystal laser rod can be produced by making single crystal as the seed crystal adhered to the tip of a bar contact molten material of about 2000 degrees Celsius contained in a melting pot, and then gradually pulling up the bar to grow the seed crystal. This pull up takes several months.

Then, a ceramic laser rod is formed by stuffing powder materials in a mold, applying a pressure to the stuffed material to mold and then, sintering as ceramics. The temperature for sintering YAG ceramics for example, is about 1750 degrees Celsius. It is possible to produce an appropriate laser rod by removing inside air bubbles during sintering.

### [Excitation light source]

The excitation light source placed in the laser emitting apparatus of the present invention is arranged parallel to the solid laser rod in general. If the excitation light source is LD (laser diode) and so forth, the excitation light source may be located on an extension of the end surface of the laser rod, so that excitation light may be incident on the end surface of the laser rod.

Since the excitation light source arranged parallel to the solid laser rod can uniformly illuminate the solid laser rod entirely with excitation light, it is possible to obtain stable laser light.

Although an excitation light source is not limited in particular as long as the excitation light source is a member that is capable of emitting light to excite a solid laser rod, the excitation light source may be a flash lamp. Preferably, the diameter of the lamp tube of the flash lamp is about 2 to 8 mm, and preferably, the flash lamp is shaped as a bar parallel to the laser rod.

This flash lamp can be produced by sealing inert gas such as xenon and krypton in a glass tube, providing electrodes at both ends of the flash lamp and forming a spark coil inside. Particularly, a flash lamp in which xenon is sealed as filler gas can provide high brightness.

### [Lens barrel]

The lens barrel located in the laser emitting apparatus encloses therein the excitation light source. The inner surface of the lens barrel reflects light from the excitation light source. The inner surface of the lens barrel reflects light to focus on the laser rod.

In order to focus light reflecting from the inner surface of the lens barrel on the laser rod, preferably the shape of the lens barrel is an oval for example, and the laser rod and the flash lamp are placed on two focal points in the oval. By this means, light reflecting from the inner surface of the lens barrel uniformly illuminates the side surface of the rod.

Naturally, the shape of the lens barrel is not limited in particular on the condition that light from the excitation light source is reflected on the inner surface of the lens barrel and is focused to illuminate the laser rod.

The inner surface of the lens barrel needs to at least reflect excitation light, and is, for example, aluminum coated with silver, high-intensity aluminum, glass coated with silver, or ceramics.

### [Reflecting mirror or reflecting film]

With the laser emitting apparatus according to the present invention, preferably, the reflecting mirror is provided on the front end and the back end of the solid laser rod parallel to the exit axis so as to amplify laser light oscillated from the solid laser rod. The reflectivity of the reflecting mirror located backward in the exit direction is adjusted to approximately total reflection equal to or more than 99.5 % while the reflectivity of the reflecting mirror located forward in the exit direction is adjusted to 80 to 95 %, so that amplified laser light is emitted through the reflecting mirror forward in the exit direction. The approximately total reflecting mirror having a reflectivity equal to or more than 99.5 %, for example, is applied aluminum mirror coating with a protective film, or is a coated surface with a low absorption dielectic multilayer film. On the other hand, the reflecting mirror having a reflectivity of 80 to 95 % is, for example, a coated surface with a low absorption dielectic multilayer film.

Meanwhile, with the laser emitting apparatus according to the present invention, both end surfaces of the solid laser rod forward and backward in the laser light exit direction may be directly coated with films to serve as reflecting mirrors instead of the reflecting mirrors arranged on the front and back ends of the sold-state laser rod. A film to serve as a reflecting mirror (referred to as "reflecting film") is, for example, a dielectric multilayer film that prevents absorption of light.

Only any one of the front and back end surfaces of the solid laser rod may be coated with a reflecting film, or both end surfaces may be coated with a reflecting film.

### [Lens]

The laser emitting apparatus according to the present invention preferably has a lens for focusing laser light emitted from the solid laser rod on a predetermined position. The lens may be made of sapphire glass, borosilicate glass, or fluoride such as calcium fluoride as long as the lens allows emitted laser light to pass through and focus without deterioration.

### [Laser transmitting plate]

The laser unit according to the present invention preferably has a laser transmitting plate for alleviating pain in front of the object to illuminate with laser light. It is preferable that one side of the laser transmitting plate has an average surface roughness Ra of 0.3 µm to 1 µm, and the other side is a mirror surface. A material for the laser transmitting plate preferably allows the laser wavelength to pass through, so as to minimize attenuation of the power of laser light. For laser light having a wavelength of 2 to 3 µm, sapphire glass, quartz, synthetic quartz, fluoride glass and so forth are preferable. When the thickness of the laser transmitting plate is about 0.1 mm to 0.3 mm, borosilicate glass may be applicable. In order to make predetermined roughness, one surface of a plate in which mirror surface processing is applied to both surfaces to make the thickness equal to or less than several nm is placed on a tin surface plate on which abrasive grains having a grain diameter of about 15 µm are dispersed so as to be wrapped in the one surface. Roughness is controlled depending on the size of abrasive grain and the material.

Although the area having predetermined roughness may be minimally only the part through which laser light passes, a part including the part through which laser light passes preferably has predetermined roughness taking into account variations in fabrication and variations in producing a part having predetermined roughness. In order to partially process the laser transmitting plate, it is preferable to mask and process parts that are not processed. At this time, etching with solution that is capable of dissolving glass makes processing easier.

### [Application of laser unit]

The laser unit according to the present invention is used to illuminate human body (a fingertip in particular) with laser light and form a puncturing hole by ablation to obtain blood for test For example, the laser unit can be used as a puncturing member for a blood test apparatus. In this case, it is preferable to emit one pulse or several pulses, and basically, it is not necessary to cool off the blood test apparatus. Nevertheless, when pulses emitted several times, it would be better to cool down the apparatus a little to maintain the performance of the apparatus.

In addition, the laser unit to draw blood from human body can efficiently emit laser light at a low applied voltage.

When the laser unit according to the present invention is used to puncture skin, preferably, the laser unit is compact (e.g. a size equal to or smaller than 15 square mm x a length of 50 mm) In addition, when the excitation light source is a flash lamp, preferably, the input voltage is 200 V to 700 V, the capacity of the charged capacitor is 200 µF to 900 µF (preferably, the input voltage is 200 V to 400 V and the capacity of the charged capacitor is 200 µF to 300 µF), and preferably, the output is 20 mJ to 120 mJ.

Preferably, the focal distance of the focusing lens is 10 mm to 35 mm, and, more preferably, is 15 mm to 20 mm. In addition, since the lens on the optical path of laser light could be contaminated with ablated materials when skin is ablated, a lens cover transparent to laser light is preferably provided, and the lens cover transparent to laser light is preferably glass having a thickness of about 0.1 to 1.0 mm or fluoroplastic such as Teflon (registered trademark). Moreover, in order to alleviate pain during laser puncturing, preferably, the cover is formed such that one side has an average surface roughness Ra of 0.3 µm to 1µm and the other side is a laser transmitting plate of a mirror surface.

### (Embodiment)

FIG.2 is a cross sectional view of a laser unit according to he present embodiment.

As shown in FIG.2, laser unit 1 is configured including ens barrel 2, laser rod 3, reflecting mirror 4, reflecting mirror 5, flash lamp 6, lens 7, power supply 8 and laser transmitting plate 9.

The inner surface of lens barrel 2 is processed to be a reflecting mirror. The reflecting mirror may be any one of aluminum coated with silver, high-intensity aluminum, glass coated with silver, ceramics, and so forth. The shape of lens barrel 2 is not limited to the shape shown in the figure, and a circle or other shapes having a curvature are applicable as long as light emitted from flash lamp 6 is reflected on the inner surface of lens barrel 2 and is focused to illuminate laser rod 3.

The shape of the cross section of lens barrel 2 is a circle or an oval. Although laser rod 3 basically has a cylindrical shape, the shape may be a prism, and also flash lamp 6 basically has a cylindrical shape.

A YAG laser (Er: YAG) doped with erbium is used as laser rod 3. To be more specific, laser rod 3 is a YAG laser (Er: YAG) doped with erbium (Er) at 50 %. Nevertheless, laser rod 3 may be solid YAG crystal doped with erbium (Er), neodymium (Nd), holmium (Ho) and so forth, and other laser rods are applicable.

Here, although a case has been described where the shape of laser rod 3 is a cylinder, other shapes are applicable. The side surface of the cylinder is roughened to a degree to prevent excitation light from reflecting. Although a case has been described where laser rod 3 is a cylinder having a diameter of 1 mm to 4 mm and a length of 10 mm to 60 mm, the size may be arbitrarily set.

Reflecting mirror 4 is placed on one end surface of laser rod 3 and reflecting mirror 5 is placed on the other end surface of laser rod 3. While the reflectivity of reflecting mirror 4 is equal to or more than 99.5 %, that is, nearly total reflection, the reflectivity of reflecting mirror 5 is adjusted to 80 % to 95 %. Aluminum mirror coating with a protective film or low absorption dielectric coating (e.g. SiO₂) is applied to reflecting mirror 4. Multilayer film dielectric coating, including zirconia, tantalum, SiO₂ and so forth, is applied to reflecting mirror 5.

Reflecting mirror 4 and reflecting mirror 5 (output mirrors) amplify the laser light oscillated from laser rod 3, the amplified laser light is emitted passing through reflecting mirror 5.

As described above, in laser unit 1, laser light amplified by stimulated emission passes through reflecting mirror 5 and then is focused on a predetermined position with lens 7. Materials for lens 7 are as described above. It is possible to alleviate pain during laser puncturing by placing the laser transmitting plate on the back end surface of the lens such that laser light passes through from the mirror surface toward the surface of predetermined roughness.

Flash lamp 6 has a lamp tube sealing therein xenon gas. The inner diameter of the lamp tube of flash lamp 6 is 2 to 8 mm. Flash lamp 6 is normally arranged parallel to laser rod 3. Here, two flash lamps 6 may be arranged to sandwich laser rod 3.

A voltage of about 5 to 10 kV is applied instantaneously to electrodes at both ends of flash lamp 6 as a trigger voltage after applying a voltage of about 200 V to 700 V. Then, the voltage of the spark coil is raised, the sealed xenon gas is ionized and electricity flows in the ionized xenon gas to emit light.

Lens 7 is located on the axis in the longitudinal direction of laser rod 3 and serves to focus laser light emitted from laser rod 3 at one point. If the focal distance of lens 7 is shorter, the laser spot diameter is smaller when laser light focuses, and if the focal distance is longer, the laser spot diameter is larger. In addition, if the focal distance is shorter, the focusing angle for focusing laser light is larger and therefore laser light concentrates on the focusing part, so that shock wave, which is the wave nature of laser light, is prone to occur. Although this shock can be reduced by extending the focal distance, the laser spot diameter is larger when the focal distance is longer, so that a large laser power is required to perform puncturing.

Power supply 8 is a power supply section to illuminate with laser light.

In laser transmitting plate 9, the surface receiving laser light is a mirror surface and the surface outputting laser light has a surface roughness (e.g. average surface roughness Ra). As described above, although sapphire glass, quartz, synthetic quartz, fluoride glass and so forth are preferable as the material for laser transmitting plate 9, borosilicate glass is also applicable. In addition, in order to make a predetermined roughness, the laser transmitting plate is produced by placing one surface of a plate having both surfaces to which mirror surface processing is applied to make the thickness equal to or less than several nm, on a tin surface plate on which abrasive grains having a grain diameter of about 15 µm are dispersed, and wrapping it in plastic.

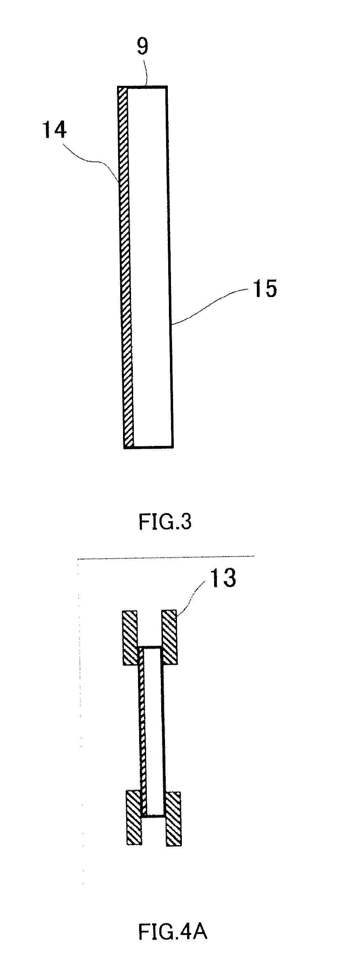

FIG.3 is a cross sectional view of the above-described laser transmitting plate 9.

As shown in FIG.3, laser transmitting section 9 has rough part 14 having a predetermined roughness and mirror surface 15, and rough part 14 has an average roughness value Ra of 0.3 µm.to 1 µm.

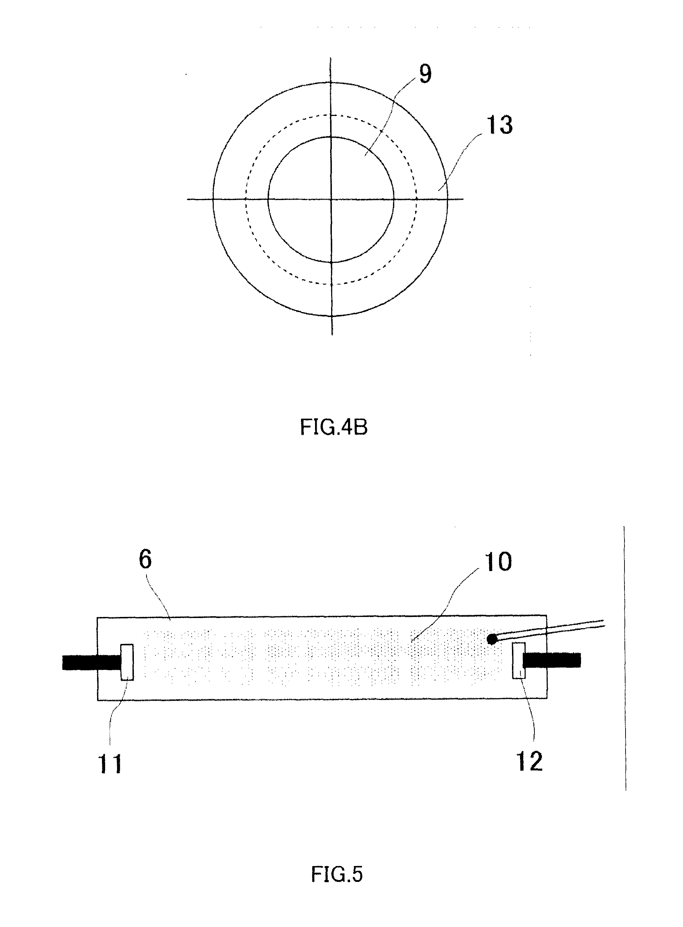

FIG.4 is a cross sectional view of the above-described laser transmitting section 9 and FIG.4B is a plan view of the above-described laser transmitting plate 9. A method of supporting laser transmitting plate 9 will be described with reference to FIG.4A and FIG.4B.

In laser transmitting plate 9, shock wave occurs on the rough surface when laser light passes through. Here, since bending stress concentrates on a part of laser transmitting plate 9 when laser transmitting plate 9 is supported with an inflexible member, the part deteriorates and then is damaged by repeatedly allowing laser light to pass through. Therefore, preferably, the material for supporting section 13 shown in FIG.4 is a flexible member such as rubber made of silicon, polyurethane and so forth, foaming agent such as styrene and a spring member such as phosphor bronze. Preferably, the flexibility of supporting section 13 is applied to the part that supports the rough part of laser transmitting plate 9 in particular. The effect is further improved by applying flexibility to both surfaces.

Now, operations of laser unit 1 configured as described above will be explained.

First, the basic operation of laser unit 1 will be explained.

As shown in FIG.2, excitation light emitted from flash lamp 6 by power supply 8 enters inside laser rod 3 and excites doped laser- active material (erbium (Er) in the present embodiment) to generate light. The generated light reflects between reflecting mirror 4 and reflecting mirror 5 in laser rod 3, and is amplified and oscillated. Part of laser light amplified by stimulated emission passes through reflecting mirror 5. The laser light having passed through reflecting mirror 5 exits through lens 7 and focuses passing through laser transmitting plate 9.

Next, operations of laser unit 1 will be explained by citing concrete examples.

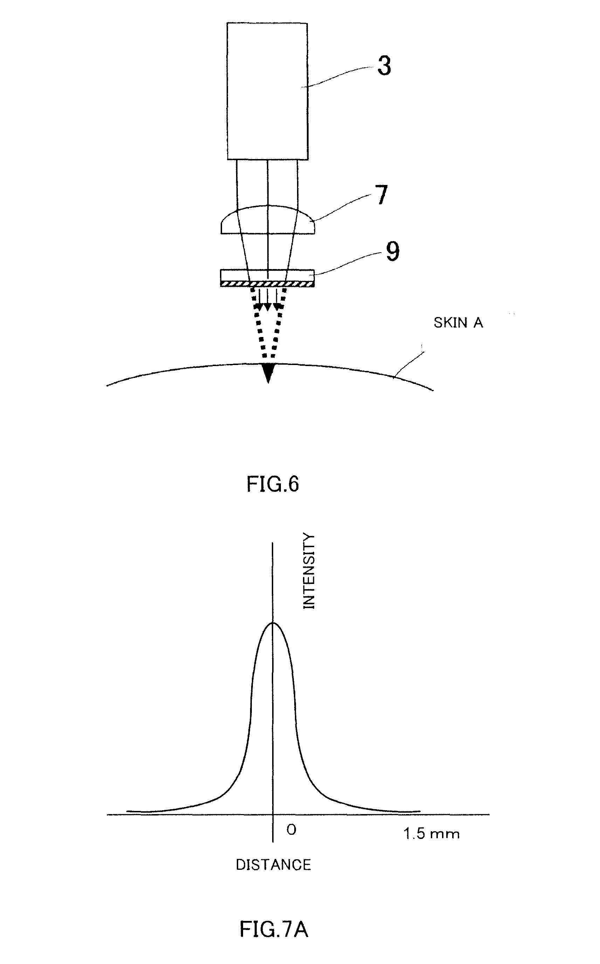

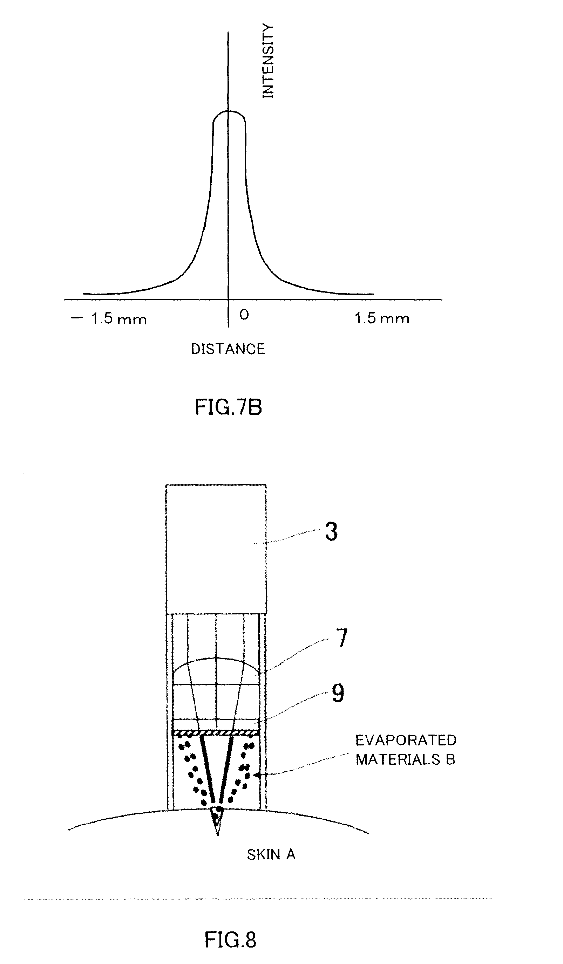

FIG.5 is a cross sectional view showing the state of flash lamp 6. FIG.6 is a drawing explaining the operating state of laser light where laser light having passed through lens 7 generates shock wave on laser transmitting plate 9 and is focused on a predetermined position. FIG.7 is a drawing explaining performance in the presence or absence of laser transmitting plate 9. FIG.8 is a drawing explaining the state of scattering of evaporated materials generated by ablation when laser light illuminates skin. Here, illustration of the circuitry for driving the apparatus is omitted.

As shown in FIG. 5, light is emitted by applying a trigger voltage of 7 kV to 9 kV between conductive film 10 attached to the tubular surface of flash lamp 6 and negative electrode 11 (cathode) in the lamp tube of flash lamp 6 and simultaneously applying a voltage of 200 V to 700 V between negative electrode 11 and positive electrode 12 in flash lamp 6.

At this time, pulsed emission is performed under the condition that the duration to apply the voltage between electrodes in flash lamp 6 is 200 µsec to 2 msec. Laser rod 3 of laser unit 1 according to the present embodiment is configured by an Er: YAG laser doped with Er at 50 %, and emitted light is taken in laser rod 3. By this means, light emitted when the Er molecules return to a stable state is amplified between reflecting mirror 4 and reflecting mirror 5 by stimulated emission, and part of the light passes through reflecting mirror 5 and is emitted.

As a result of this, laser light having a wavelength of 2.94 µm is emitted from (exits) laser unit 1 according to the present embodiment. The laser emission can pass through lens 7 having a focal distance 25 mm, generate shock wave through laser transmitting plate 9, and then focus on a position 25 mm apart from the tip of lens 7.

As shown in FIG.6, laser light is incident on mirror section 15 of laser transmitting plate 9 and generates the shock wave indicated by the arrow when passing through the surface of rough part 14. At the same time, the incident light is slightly scattered on the part indicated by the dashed line, so that pain of the punctured part such as a finger can be reduced.

The profile of the emitted laser light may be viewed using a profiler: RYROCAM III manufactured by Ophir Optronics Ltd.

FIG.7 is drawing explaining the performance at the presence or absence of laser transmitting plate 9, and here, the vertical axis shows the intensity and the horizontal axis shows the distance.

FIG.7A shows a case of the absence of laser transmitting plate 9, and FIG.7B shows a case of the presence of laser transmitting plate 9. Data can be obtained as shown in FIG.7.

In the case of the absence of laser transmitting plate 9, the intensity of laser light is concentrated at the center as shown in FIG.7A, but when laser transmitting plate 9 is added, it can be observed that the concentration of intensity at the center is decreased as shown in FIG.7B.

As a result of this, pain during puncturing is alleviated In addition, skin A is punctured with laser light as shown in FIG.8, evaporated materials B generated by ablation scatter toward the lens. Laser transmitting plate 9 also has a function to prevent the lens from being contaminated with these evaporated materials B. To realize this function, laser transmitting plate 9 has a structure not having a gap between laser transmitting plate 9 and the casing holding the lens so as not to allow evaporated materials to pass through.

Although laser transmitting plate 9 may be located between lens 7 and skin A as shown in FIG.6, the loss of laser light at the time laser light passes through laser transmitting plate 9 because the scale of laser light passing through differs depending on locations in laser transmitting plate 9, and the performance of laser light is likely to vary due to variations in fabrication. However, laser transmitting plate 9 is located between lens 7 and the punctured site, so that an effect is provided that it is possible to protect lens 7 from evaporated materials generated when the punctured spot is ablated with laser light.

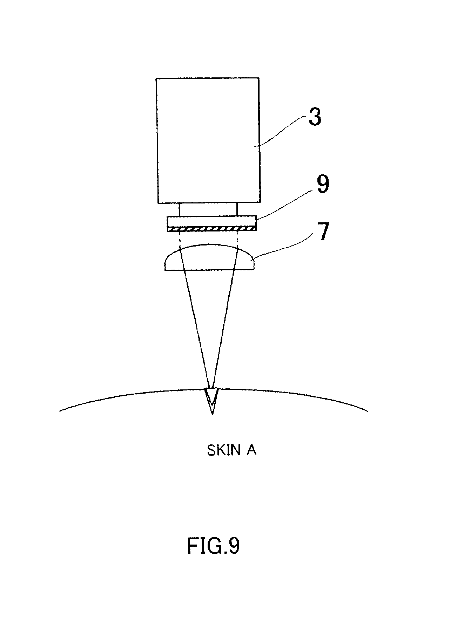

FIG.9 is a configuration diagram of the laser unit in which laser transmitting plate 9 is located between lens 7 and laser rod 3.

As shown in FIG.9, laser transmitting plate 9 is placed between laser rod 3 and lens 7 in the laser unit. Since the scale of laser light passing through does not differ depending on where laser transmitting plate 9 is placed, an effect is provided that the loss of laser light when laser light passes through laser transmitting plate 9 does not vary. However, the same effect of alleviating pain during puncturing can be alleviated is provided in both the configurations shown in FIG.6 and FIG.9.

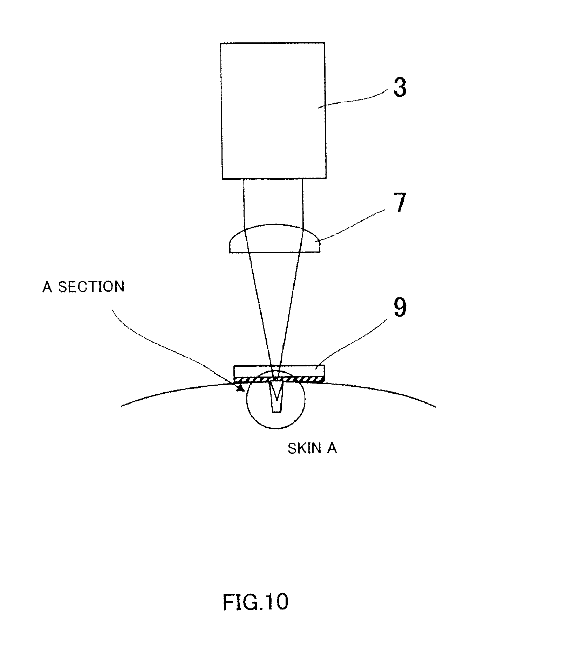

FIG.10 is a configuration diagram of the laser unit when laser transmitting plate 9 is placed in a position where laser transmitting plate 9 contacts skin.

As for positioning of laser transmitting plate 9, the part with predetermined roughness may directly contact the punctured spot as shown in FIG.10. In this case, materials evaporated by laser light ablation adhere to the laser transmitting plate Therefore, it is necessary to provide a mechanism for wiping off adhered evaporated materials or to replace the laser transmitting plate each time. As described above, when laser transmitting plate 9 directly contacts skin, which is the object to puncture, the refractive index of light incident on skin A through laser transmitting plate 9 is lower than in a case in which there is the air between skin A and laser transmitting plate 9, so that laser light is refracted little as shown in section A of FIG.10 and penetrates into skin. Therefore, pain during puncturing with laser light is further alleviated.

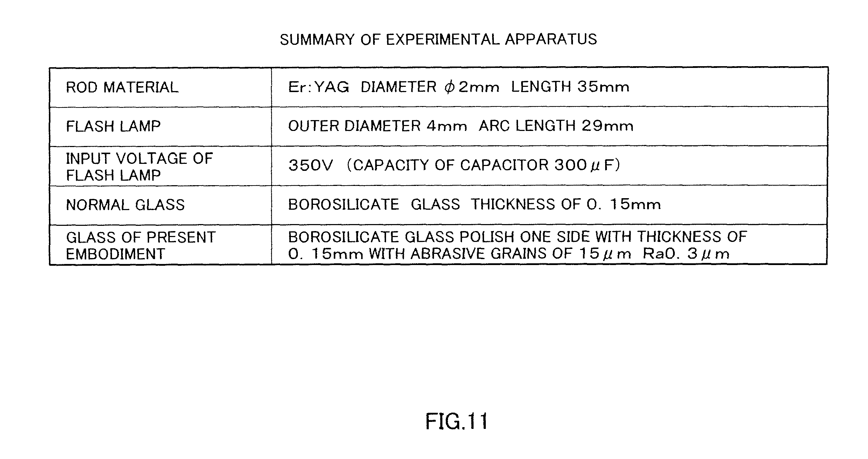

FIG.11 is a table showing a summary of an experimental apparatus applied to laser unit 1 according to the present embodiment. As shown in FIG.11, the material of laser rod 3 is Er: YAG, and the diameter is Φ2 mm and the length is 35 mm. Flash lamp 6 has an outer diameter of 4 mm, an arc length of 29 mm, and an input voltage of 350 V (the capacity of the capacitor is 300 µF). Laser transmitting plate 9 is produced using borosilicate glass with a thickness of 0.15 mm, and the surface in the side through which laser light exits is produced to have an average surface roughness Ra of 0.3 µm. Rough part 14 of laser transmitting plate 9 is produced by grinding with abrasive grains of 15 µm.

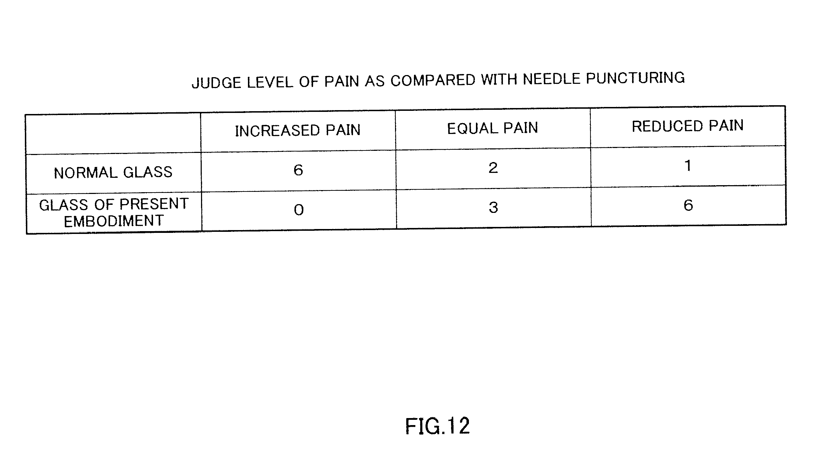

FIG.12 is a drawing showing the degree of pain during puncturing with the laser unit 1 produced according to the experimental apparatus of FIG.11, as compared with needle puncturing. The number of subjects is nine (adult males). First, with a conventional apparatus using normal glass, the number of people who reported increased pain compared to needle puncturing was six, the number of people who reported equal pain compared to needle puncturing was two and the number of people who reported reduced pain compared to needle puncturing was one. On the other hand, with laser transmitting plate 9 according to the present embodiment, people who reported increased pain compared to needle puncturing were zero, the number of people who reported equal pain compared to needle puncturing was three and the number of people who reported reduced pain compared to needle puncturing was six. In particular, although, with the conventional apparatus, the number of people who reported increased pain compared to needle puncturing was six, the number was zero with the present embodiment. On the other hand, although, with the conventional apparatus, the number of people who reported reduced pain compared to needle puncturing was one, the number was six with the present embodiment. According to this judgment result, it can be assured that the level of pain with laser unit 1 according to the present embodiment is clearly lower than that of the conventional apparatus.

With the conventional apparatus, the cause to feel pain is that energy not used for evaporation penetrates into a deep part of skin and stimulates the pain spot. With the present embodiment, rough part 14 of laser transmitting plate 9 is so-called frosted glass produced by grinding glass with abrasive grains. This frosted glass is inserted between laser light and skin to disperse laser light adequately and therefore an effect of reducing pain can be provided. In addition, as shown in FIG.7B, it was experimentally assured that laser light was scattered at the center of laser light and therefore the concentration of intensity at the center is decreased.

Moreover, when laser light passes through rough part 14 from the mirror section 15 side in laser transmitting plate 9, shock wave is generated in rough part 14. At this time, since energy in the form of the shock wave is released in advance, pain in skin and so forth is reduced.

As described in detail above, according to the present embodiment, laser unit 1 has laser rod 3 that emits laser light; lens 7 that focuses laser light emitted from emitting section; and laser transmitting plate 9 in which the surface receiving laser light is a mirror section 15 and the surface outputting laser light has rough part 14 with an average surface roughness Ra. By this configuration, the laser light having passed through laser transmitting plate 9 scatters on rough part 14, and therefore the intensity of laser light illuminating skin is not focused at the center. In addition, energy is released in advance by generating shock wave when laser light passes through rough part 14. These factors can realize laser unit 1 being capable of reducing pain when the object to illuminate is skin (a finger in particular) and so forth.

The above descriptions are illustrations of the preferred embodiments of the present invention and the scope of the invention is not limited to these.

Moreover, although the name "laser unit" is used in each above-described embodiment for convenience of explanation, the name of the apparatus may be "laser apparatus," "puncturing apparatus," and so forth.

Moreover, the type, the number, the connection method and so forth of each component constituting the above-described laser unit, for example a laser rod, are not limited.

The disclosure of Japanese Patent Application No.2007-313273, filed on December 4, 2007, including the specification, drawings and abstract, is incorporated herein by reference in its entirety.

The laser unit according to the present invention is applicable to a blood analysis apparatus that samples blood from skin with a blood sampling means such as a laser unit and analyzes blood components.

## Claims

1. A laser unit comprising:
an emitting section that emits laser light;
a lens that focuses laser light emitted from the emitting section;
a laser transmitting plate placed on an optical path for emitting laser light, a surface receiving the laser light being a mirror surface, and a surface outputting_ the laser light having predetermined roughness.

2. The laser unit according to claim 1, wherein:
a wavelength of the laser light emitted from the emitting section is 2 to 3 µm; and
an average value of the predetermined roughness Ra of one surface of the laser light transmitting plate is 0.3 µm to 1 µm.

3. The laser unit according to claim 1, wherein in the surface outputting the laser light in the laser transmitting plate, only a part through which the laser light passes, or a portion including the part through which the laser light passes, is formed with the predetermined roughness.

4. The laser unit according to claim 1, wherein the part of the predetermined roughness in the laser transmitting plate can contact an object to illuminate.

5. The laser unit according to claim 1, wherein:
the wavelength of the laser light emitted from the emitting section is 2 to 3 µm and a thickness of the laser transmitting plate is 0.1 to 0.3 mm.

6. The laser unit according to claim 1, wherein the laser transmitting plate is supported by a member with a damper.

7. The laser unit according to claim 1, wherein the laser transmitting plate is placed between the lens and the object to illuminate.

8. The laser unit according to claim 1, wherein the laser transmitting plate is placed between the emitting section and the lens.
